(19) Europäisches Patentamt
European Patent Office
Office européen des brevets

(11) Numéro de publication : **0 309 364 B1**

# (12) FASCICULE DE BREVET EUROPEEN

(45) Date de publication du fascicule du brevet : **22.04.92 Bulletin 92/17**

(51) Int. Cl.$^5$ : **C07C 275/62,** C07C 271/06

(21) Numéro de dépôt : **88420312.6**

(22) Date de dépôt : **12.09.88**

## (54) Procédé de purification et d'isolement de condensats d'isocyanates par extraction au co2 liquide ou supercritique.

(30) Priorité : **24.09.87 FR 8713478**

(43) Date de publication de la demande : **29.03.89 Bulletin 89/13**

(45) Mention de la délivrance du brevet : **22.04.92 Bulletin 92/17**

(84) Etats contractants désignés : **BE CH DE ES FR GB IT LI LU NL SE**

(56) Documents cités :
**CHEMISTRY AND INDUSTRY, no. 12, 19 juin 1982, pages 394-396, Londres, GB; R. BOTT: "Fundamentals of carbon dioxide in solvent extraction"**

(73) Titulaire : **RHONE-POULENC CHIMIE**
**25, quai Paul Doumer**
**F-92408 Courbevoie Cédex (FR)**

(72) Inventeur : **Blind, André**
**10, rue Martin Basse**
**F-69300 Caluire (FR)**
Inventeur : **Collas, Gérard**
**406, avenue du 8 Mai**
**F-69300 Caluire (FR)**
Inventeur : **Robin, Jean**
**21, rue Duguesclin**
**F-69006 Lyon (FR)**
Inventeur : **Sagi, Ferenc**
**133, rue Ferdinand Buisson**
**F-69003 Lyon (FR)**

(74) Mandataire : **Dubruc, Philippe et al**
**RHONE-POULENC CHIMIE Direction de la Propriété Industrielle 25, Quai Paul Doumer**
**F-92408 Courbevoie Cedex (FR)**

**EP 0 309 364 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne l'isolement et la purification de condensats d'isocyanates à groupes NCO libres, obtenus à partir de di- ou polyisocyanates aromatiques, c'est à dire dont l'un au moins des groupes NCO est lié directement à un cycle aromatique.

De tels condensats sont utilisés pour préparer des mousses, des élastomères, des adhésifs, des peintures et des vernis, dont les propriétés exceptionnelles sont maintenant bien connues.

Ces condensats à groupes NCO libres sont obtenus en faisant réagir au moins un composé possédant au moins deux groupements réactifs vis à vis des isocyanates, avec un excès molaire de di- ou polyisocyanate, éventuellement au sein d'un solvant inerte vis à vis des groupes NCO. Le composé à groupements réactifs vis à vis des groupes NCO peut comporter comme sites réactifs, des groupements -OH, $-NH_2$, -SH, -COOH, -NH, $-CONH_2$, -CO-NH- , les groupements au sein dudit composé peuvent bien entendu être identiques ou différents. On peut citer comme exemples de tels composés : les diols, les polyols, les amino-alcools, les di- et polyamines. Le composé portant les groupes réactifs vis-à-vis des groupements NCO peut être constitué par un biradical aliphatique, cyclique, cycloaliphatique ou aromatique ; il peut aussi résulter lui-même de la condensation de molécules simples et conduire à un bi-radical comportant, le cas échéant, des hétéroatomes dans la chaîne. De tels condensats peuvent être :

– des polyesters issus de l'estérification d'un ou plusieurs di ou polyols par un ou plusieurs di- ou polyacides, ou encore par réaction d'une lactone cyclique sur une molécule di- ou polyfonctionnelle comportant des groupements -OH, $-NH_2$, -NHR, par exemple ;
– des polyéthers, issus de la condensation d'oxydes cycliques (oxyde d'éthylène, de propylène, de butylène, de tétraméthylène) sur une molécule di- ou polyfonctionnelle comportant des groupes -OH, $-NH_2$, NH ;
– des condensats mixtes comportant des segments polyéther et polyester.

Ces composés sont mis à réagir avec un excès de di- ou polyisocyanate, éventuellement au sein d'un solvant non réactif avec les groupes isocyanates. Comme di- ou polyisocyanates, on peut citer à titre d'exemple :

– le diisocyanato-1,3 ou -1,4 benzène,
– le diisocyanato-2,4 ou -2,6 ou -2,5 toluène (ou alkyl benzène),
– le diisocyanato-4,4'diphénylméthane, pur ou renfermant les isomères 2,4' et 2,2'.

Dans les condensats visés, le plus fréquemment employé est le diisocyanate de toluylène, soit sous forme de l'isomère 2,4 pur, soit en mélange 80/20 ou 65/35 avec l'isomère 2,6(TDI). On peut aussi utiliser des mélanges de di- ou polyisocyanates.

La réaction du polyisocyanate en excès avec le composé antagoniste est réalisée selon les moyens connus, c'est à dire en chauffant le mélange des deux produits, éventuellement en présence d'un catalyseur et/ou d'un solvant.

Un autre type de condensats, auquel référence doit être faite, est celui connu sous le nom de biuret, et possédant la formule théorique (I) ci-après :

(I)

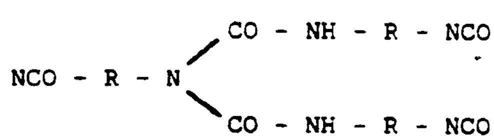

dans laquelle R représente le reste d'un diisocyanate aromatique.

De tels condensats sont obtenus par réaction d'eau avec un excès de diisocyanate ; l'eau peut être utilisée telle quelle, avec ou sans solvant ; ou peut aussi remplacer l'eau par d'autres agents de biurétisation, tels les alcools tertiaires, les amines, l'acide formique et l'hydrogène sulfuré, notamment.

Un autre type de condensats, auquel référence doit également être faite, peut être obtenu par cyclotrimérisation partielle de diisocyanate, sous l'influence de catalyseurs basiques ou organométalliques, le cas échéant au sein d'un solvant. On obtient ainsi un isocyanurate possédant la formule théorique (II) ci-après :

(II) 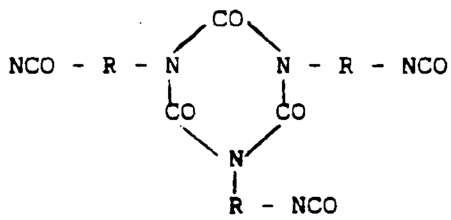

dans laquelle R a la signification donnée précédemment.

Les catalyseurs pouvant être utilisés sont décrits par exemple dans Russian Chem. Rev 41 (9) 1972, pages 776 et 777, ou dans "Newer Methods of Preparative Organic Chemistry" Vol VI, 1971, pages 280 à 284.

Le point commun de tous ces condensats à NCO libres est de renfermer, en fin de réaction, une quantité plus ou moins importante du diisocyanate utilisé en excès. Il est souvent nécessaire d'éliminer ce diisocyanate du polycondensat réalisé, notamment en raison de la toxicité due à la volatilité dudit diisocyanate. En effet, lors de la mise en oeuvre de tels précondensats obtenus avec un diisocyanate comme le TDI, la présence d'une quantité plus ou moins importante de TDI peut présenter des risques pour l'utilisation du précondensat ; ces risques sont très grands lorsque le précondensat est mis en oeuvre en couche mince, comme cela est le cas pour les peintures et vernis, par exemple, car l'émission de vapeurs de TDI toxique peut devenir importante et dépasser les limites autorisées légalement dans l'atmosphère environnante.

Une autre raison peut résider dans la mise en oeuvre de tels précondensats, notamment de ceux issus de la réaction de polyesters ou polyéthers avec un excès de diisocyanate (ces précondensats étant le plus souvent dénommés prépolymères), pour la fabrication de matériaux élastomériques cellulaires ou non. Dans ce cas, ledit prépolymère est mis à réagir avec une quantité stoechiométrique d'un réactif au moins difonctionnel et comportant des groupes réactifs avec les NCO, au sens indiqué précédemment. Le matériau final comportera une quantité plus ou moins grande du produit de condensation du diisocyanate en excès avec le réactif difonctionnel, dont la présence peut être préjudiciable aux propriétés du matériau élastomérique final. Dans ce cas, l'élimination du diisocyanate libre sera la seule manière d'avoir un prépolymère ayant à la fois un faible degré de condensation et une basse teneur en diisocyanate libre.

L'élimination du diisocyanate en excès peut se faire par des moyens connus comme l'évaporation ou l'extraction par un solvant du diisocyanate, non solvant du condensat. Toutefois, l'évaporation nécessite l'emploi de températures élevées qui sont préjudiciables à la qualité du précondensat. Cet inconvénient se manifeste de manière d'autant plus importante que l'isocyanate utilisé est aromatique, car les NCO aromatiques sont très réactifs et conduisent, par des réactions secondaires à chaud, à un produit de viscosité élevée, voire résineux, pouvant bloquer l'évaporateur ; de plus, un bon épuisement du diisocyanate libre nécessite l'emploi d'évaporateurs à film mince agités ; ce sont des appareils onéreux et qui nécessitent, dans ce cas, des arrêts et nettoyages fréquents, car le plus souvent le condensat fondu se résinifie peu à peu dans l'appareil. D'autre part, l'extraction par un solvant du diisocyanate non solvant du condensat (comme l'hexane, l'octane...) est longue et malaisée ; en effet, dès l'addition du non solvant, le condensat tend à précipiter sous forme d'une masse collante de laquelle il est difficile d'extraire la totalité du diisocyanate monomère libre.

On a trouvé que l'opération de séparation du condensat à NCO libre d'avec l'excès de diisocyanate libre est facile à réaliser par traitement du condensat brut par du gaz carbonique à l'état liquide ou supercritique. Le gaz carbonique dont l'usage est préconisé est bon marché, non toxique et ininflammable. On a constaté que, non seulement le $CO_2$ supercritique dissout et extrait le diisocyanate libre présent dans le condensat, mais aussi que le condensat, au sein du $CO_2$ liquide ou supercritique, reste liquide quand bien même il a une consistance solide ou résineuse à la température d'extraction, ce qui permet ensuite de véhiculer et de dissoudre commodément, et à température modérée, le condensat débarrassé de son diisocyanate libre.

Cette extraction peut se faire en continu ou en discontinu à une température supérieure à 31,4°C (température critique du $CO_2$) et à une pression comprise entre 7 300 et 50 000 KPa. De préférence, la température est comprise entre 31,4°C et 100°C et la pression entre 7 300 et 35 000 KPa. Ceci concerne le $CO_2$ à l'état supercritique. A l'état liquide, le $CO_2$ est mis en oeuvre à une température comprise entre 0 et 31°C, et sous une pression de 3 000 à 50 000 KPa. Il est souvent préférable d'opérer entre 30 et 31°C lorsque la viscosité du condensat est importante. La pression peut aller de 6 000 à 30 000 KPa.

Le condensat renfermant le diisocyanate libre est traité par le $CO_2$ liquide ou à l'état supercritique ; on peut opérer en discontinu, c'est à dire en mélangeant dans un réacteur le condensat à NCO libre à purifier avec le $CO_2$ à l'état liquide ou supercritique.

Il est possible de traiter la masse réactionnelle dès la fin de la réaction de condensation.

Cependant, compte tenu des quantités importantes de diisocyanate monomère en excès et le plus fréquemment du ou des solvants, il peut être préférable et plus économique de procéder préalablement à une élimination rapide de la plus grande partie du diisocyanate monomère en excès et du solvant lorsque ce dernier est présent.

Ce traitement, qui est souvent une évaporation très rapide sous pression réduite, ne nécessite pas de chauffer à une température élevée. Il ne présente donc pas, ou très peu, l'inconvénient mentionné précédemment, c'est-à-dire de provoquer dans l'appareillage un dépôt polymérique nécessitant de fréquents arrêts pour nettoyage.

En pratique donc, la masse obtenue après la réaction de condensation est envoyée dans un évaporateur à couche mince, qui permet de séparer la majeure partie du solvant et une partie notable du diisocyanate monomère en excès.

Le polyisocyanate obtenu, contenant une quantité encore relativement importante de diisocyanate monomère et éventuellement des traces de solvant, est alors traité à l'aide de $CO_2$ liquide ou de $CO_2$ à l'état supercritique.

Après séparation du gaz carbonique renfermant le diisocyanate monomère et éventuellement du solvant, d'avec le condensat purifié, on peut séparer le $CO_2$ d'avec les produits extraits par détente et/ou augmentation de température.

L'extraction peut être conduite de manière conventionnelle, dans un appareillage connu en soi.

A partir d'une source de gaz carbonique (gaz d'extraction), on envoie ledit gaz dans un échangeur thermique où il est liquéfié ; il est ensuite véhiculé à la pression désirée à l'aide d'une pompe vers un autre échangeur thermique où il est porté à la température choisie pour l'extraction.

Le gaz d'extraction à l'état liquide ou supercritique est ensuite envoyé dans l'appareil d'extraction qui peut être par exemple une colonne remplie d'un garnissage permettant un meilleur contact entre le condensat avec le gaz d'extraction. Le condensat peut être introduit à l'autre extrémité de la colonne : on a alors une extraction à contre-courant. Moins fréquemment, on peut l'introduire à la même extrémité que le gaz d'extraction : on a alors une extraction à co-courant. Le condensat purifié est récupéré à une extrémité de la colonne d'extraction, tandis que le gaz d'extraction chargé du diisocyanate monomère, et éventuellement de solvants, est traité pour le séparer des produits extraits.

On peut pour cela agir soit par diminution de sa pression soit par augmentation de sa température, soit à la fois par diminution de sa pression et augmentation de sa température. Ces conditions ont pour but de modifier le pouvoir solvant du gaz d'extraction.

La diminution de pression ou détente, peut se faire en une ou plusieurs étapes et le gaz d'extraction peut être détendu jusqu'à une pression égale à la pression atmosphérique ou jusqu'à une pression plus élevée, sous laquelle il sera recyclé dans le cas d'un procédé continu.

En effet, si le gaz d'extraction est recyclé, il est économiquement préférable de ne pas le détendre jusqu'à la pression atmosphérique, ce qui nécessiterait une plus grande dépense d'énergie pour le recomprimer dans le cycle suivant le procédé. Il est préférable de le détendre jusqu'à une pression sous laquelle les composés extraits ne sont pas, ou sont très peu, solubles.

Le condensat, liquéfié par le gaz d'extraction sous pression peut être véhiculé et détendu dans un récipient de capacité suffisante dans lequel le gaz détendu pourra s'échapper, laissant sur les parois le condensat sous forme de poudre ou de liquide visqueux ; ou encore, le condensat liquéfié par le gaz d'extraction peut être reçu dans un solvant ou dans un mélange de solvants ce qui donne après détente, une solution de condensat prête à l'emploi.

Comme cela a été indiqué précédemment, le procédé peut être mis en oeuvre de manière continue ou discontinue, et l'appareillage utilisé n'est pas limité à la description du principe de fonctionnement décrit ci-avant.

Généralement, dans le cadre du procédé de l'invention on préfère mettre en oeuvre le gaz d'extraction à l'état supercritique.

Les exemples qui suivent illustrent la présente invention.

EXEMPLE I :

Dans un ballon tricol de 2 litres, muni d'une agitation, d'un réfrigérant, d'un chauffage par bain d'huile et d'une ampoule de coulée, on charge 1044g de TDI (Toluène diisocyanate, mélange 80/20 des isomères 2,4 et 2,6) et 210g d'acétone.

On porte à 45°C et coule en 1 heure une solution de 14,4g d'eau dans 40 g d'acétone, avec le réfrigérant en position de reflux. On met alors le réfrigérant en position de distillation (condensation extérieure) et on monte à 145°C en 3 heures. On maintient 1 heure à 145°C avec un balayage d'azote, qui élimine la majorité de l'acétone restant.

On obtient (après refroidissement) une solution de TDI biuret dans du TDI en excès, titrant 0,940 groupements NCO pour 100 g. Cette solution est constituée de 2/3 de TDI libre et 1/3 de TDI biuret et ses homologues supérieurs.

Extraction n°1 : ($CO_2$ liquide)

Dans un réacteur de 50 $cm^3$ résistant à la pression, on charge 26,65 g de la solution TDI + TDI biuret obtenue ci-dessus.

On envoie le $CO_2$ à 8 000 kPa et à 20°C, avec un débit de 1kg/heure.

Après 45 minutes, on a extrait (après élimination du $CO_2$) 14,14 g de produit constitué par 92 % de TDI et 8 % de biuret.

Après une deuxième période de 45 minutes, on extrait encore (après élimination du $CO_2$) 1,35 g de produit constitué par 97 % de TDI et 3 % de biuret.

Le produit restant peut être soutiré du réacteur : en effet il est (sous pression) sous forme d'un liquide visqueux.

Lors de la détente dans un flacon, on obtient une poudre blanche légèrement jaune, renfermant 10 à 12 % de TDI libre.

Extraction n°2 : ($CO_2$ supercritique)

On effectue, sur 27,48 g du même produit que pour l'extraction n°1, et dans le même appareil, une extraction par $CO_2$ à 50°C et sous 120-190 bars, de la manière suivante :

– 45 minutes sous 12 000 KPa (débit $CO_2$ =1 Kg/heure) fournissant après détente 11,17 g de produit extrait.
– 48 minutes sous 19 000 KPa (débit $CO_2$ =1 Kg/heure) donnant après détente 4,43 g de produit extrait.
– 15 minutes sous 19 000 KPa (débit $CO_2$ =1 Kg/heure) donnant après détente 0,1 g de produit extrait.

Le poids total des extraits est de 15,7 g, renfermant 95 % de TDI et 5 % de biuret.

Le produit restant, détendu dans un flacon, s'y dépose sous forme d'une poudre. On obtient ainsi 8,5 g de solide renfermant 5 % de TDI et 95 % de biuret.

EXEMPLE 2 :

Dans un réacteur, muni d'une agitation, d'un chauffage par bain d'huile, on charge 200g de polyester et 34,8 g de TDI (Toluène diisocyanate, mélange 80/20 des isomère 2,4 et 2,6). Le polyester est un polyadipate d'éthylène glycol, à terminaisons hydroxylées, de poids moléculaire 2000, renfermant 0,100 OH/100g.

Après chauffage de 1h30 à 80-90°C, on obtient un prépolymère à NCO libres titrant 3 % de TDI monomère libre.

Dans l'appareil d'extraction décrit dans l'exemple 1, on met 23,5 g de prépolymère et on envoie le $CO_2$ à 19 000 KPa à 50°C pendant 45 minutes au débit de 1Kg/heure.

Le résidu obtenu après détente du $CO_2$, titre 0,5 % de TDI libre.

Une extraction ext faite sur 38,5 g de prépolymère, le $CO_2$ étant à 78°C sous 18 600 KPa (débit 1 Kg/heure) pendant 55 minutes.

Après détente, le prépolymère extrait titre moins de 1000ppm de TDI libre.

## Revendications

1. Procédé de purification et d'isolement de condensats d'isocyanates à groupes NCO libres obtenus à partir de di- ou polyisocyanates aromatiques en excès et d'au moins un composé antagoniste possédant au moins deux groupements réactifs vis-à-vis des groupes NCO, ledit composé antagoniste pouvant être un diisocyanate aromatique, caractérisé en ce que lesdits condensats bruts et à groupes NCO libres sont traités par du gaz carbonique à l'état liquide ou supercritique.

2. Procédé selon la revendication 1, caractérisé en ce que le condensat est préparé à partir d'au moins un diisocyanate monomère choisi dans le groupe comprenant :

– le diisocyanato-1,3 benzène
– le diisocyanato-1,4 benzène
– le diisocyanato-2,4 toluène
– le diisocyanato-2,6 toluène
– le diisocyanato-2,5 toluène

– le diisocyanato-4,4′ diphénylméthane
– le diisocyanato-2,4′ diphénylméthane
– le diisocyanato-2,2′ diphénylméthane.

3. Procédé selon la revendication 1 ou 2, caractérisé en ce que le condensat est préparé à partir de diisocyanato-2,4 toluène seul ou en mélange avec le diisocyanato-2,6 toluène.

4. Procédé selon l'une quelconque des revendications précédentes, caractérisé en ce que le composé antagoniste utilisé pour préparer le condensat est l'eau.

5. Procédé sel on l'une quel conque des revendications 1 à 3, caractérisé en ce que le composé antagoniste utilisé pour préparer le condensat à groupes NCO libres est lui-même un condensat du type des polyesters, du type des polyéthers ou de type mixte polyéther-polyester.

6. Procédé selon l'une quelconque des revendications précédentes, caractérisé en ce que l'on opère à une température comprise entre 0 et 31°C et sous une pression de 3000 à 50 000 KPa.

7. Procédé selon la revendication 6, caractérisé en ce que l'on opère à une température comprise entre 20 et 31°C et sous une pression de 6000 à 30 000 KPa.

8. Procédé selon l'une quelconque des revendication 1 à 5, caractérisé en ce que l'on opère à une température supérieure à la température critique du gaz carbonique de préférence inférieure à 100°C et sous une pression de 7300 à 50 000 KPa et de préférence de 7300 à 35 000 KPa.

9. Procédé selon l'une quelconque des revendications précédentes, caractérisé en ce que l'on élimine, préalablement à l'extraction par le gaz carbonique à l'état liquide ou supercritique, la plus grande partie de l'excès de diisocyanate monomère et du solvant lorsque ce dernier est présent.

## Patentansprüche

1. Verfahren zum Reinigen und Isolieren von Kondensationsprodukten von Isocyanaten mit freien NCO-Gruppen, erhalten ausgehend von überschüssigen aromatischen Di- oder Polyisocyanaten und mindestens einer antagonistischen Verbindung, die mindestens zwei gegenüber den NCO-Gruppen reaktionsfähige Gruppen aufweist, wobei die antagonistische Verbindung ein aromatisches Diisocyanat sein kann, dadurch gekennzeichnet, daß die rohen und freie NCO-Gruppen enthaltenden Kondensationsprodukte mit flüssigem oder überkritischem Kohlendioxid behandelt werden.

2. Verfahren nach Anspruch 1, dadurch gekennzeichnet, daß das Kondensationsprodukt ausgehend von mindestens einem monomeren Diisocyanat aus der Gruppe:

– 1,3-Diisocyanato-benzol
– 1,4-Diisocyanato-benzol
– 2,4-Diisocyanato-toluol
– 2,6-Diisocyanato-toluol
– 2,5-Diisocyanato-toluol
– 4,4′Diisocyanato-diphenylmethan
– 2,4′-Diisocyanato-diphenylmethan
– 2,2′-Diisocyanato-diphenylmethan hergestellt wird.

3. Verfahren nach Anspruch 1 oder 2, dadurch gekennzeichnet, daß das Kondensationsprodukt ausgehend von 2,4-Diisocyanatotoluol alleine oder im Gemisch mit 2,6-Diisocyanato-toluol hergestellt wird.

4. Verfahren nach einem der vorangehenden Ansprüche, dadurch gekennzeichnet, daß die zur Herstellung des Kondensationsproduktess eingesetzte antagonistische Verbindung Wasser ist.

5. Verfahren nach einem der Ansprüche 1 bis 3, dadurch gekennzeichnet, daß die zur Herstellung des Kondensationsproduktes mit freien NCO-Gruppen eingesetzte antagonistische Verbindung selbst ein Kondensationsprodukt vom Typ Polyester, vom Typ Polyether oder vom Mischtyp Polyether-Polyester ist.

6. Verfahren nach einem der vorangehenden Ansprüche, dadurch gekennzeichnet, daß man bei einer Temperatur im Bereich von 0 bis 31°C und unter einem Druck von 3000 bis 50 000 kPa arbeitet.

7. Verfahren nach Anspruch 6, dadurch gekennzeichnet, daß man bei einer Temperatur im Bereich von 20 bis 31°C und unter einem Druck von 6000 bis 30 000 kPa arbeitet.

8. Verfahren nach einem der Ansprüche 1 bis 5, dadurch gekennzeichnet, daß man bei einer Temperatur oberhalb der kritischen Temperatur des Kohlendioxids, vorzugsweise unterhalb 100°C, und unter einem Druck von 7300 bis 50 000 kPa und vorzugsweise von 7300 bis 35 000 kPa arbeitet.

9. Verfahren nach einem der vorangehenden Ansprüche, dadurch gekennzeichnet, daß man vor der Extraktion mit flüssigem oder überkritischem Kohlendioxid den Hauptteil des überschüssigen monomeren Diisocyanats und des Lösungsmittels, wenn dieses vorhanden ist, eliminiert.

## Claims

1. Process for the purification and isolation of isocyanate condensates containing free NCO groups obtained from aromatic di- or polyisocyanates in excess and from at least one antagonist compound containing at least two groups which are reactive towards NCO groups, it being possible for the said antagonist compound to be an aromatic diisocyanate, characterised in that the said crude condensates containing free NCO groups are treated with carbon dioxide in the liquid or supercritical state.

2. Process according to claim 1, characterised in that the condensate is prepared from at least one diisocyanate monomer chosen from the group comprising:

- 1,3-diisocyanatobenzene
- 1,4-diisocyanatobenzene
- 2,4-diisocyanatotoluene
- 2,6-diisocyanatotoluene
- 2,5-diisocyanatotoluene
- 4,4′-diisocyanatodiphenylmethane
- 2,4′-diisocyanatodiphenylmethane, and
- 2,2′-diisocyanatodiphenylmethane.

3. Process according to claim 1 or 2, characterised in that the condensate is prepared from 2,4-diisocyanatotoluene by itself or mixed with 2,6-diisocyanatotoluene.

4. Process according to any one of the preceding claims, characterised in that the antagonist compound employed for preparing the condensate is water.

5. Process according to any one of claims 1 to 3, characterised in that the antagonist compound employed for preparing the condensate containing free NCO groups is itself a condensate of the polyester type, of the polyether type or of the mixed polyether-polyester type.

6. Process according to any one of the preceding claims, characterised in that the operation is carried out at a temperature of between 0 and 31°C and at a pressure of 3,000 to 50,000 kPa.

7. Process according to claim 6, characterised in that the operation is carried out at a temperature of between 20 and 31°C and a pressure of 6,000 to 30,000 kPa.

8. Process according to any one of claims 1 to 5, characterised in that the operation is carried out at a temperature above the critical temperature of carbon dioxide, preferably below 100°C, and a pressure of 7,300 to 50,000 kPa and preferably from 7,300 to 35,000 kPa.

9. Process according to any one of the preceding claims, characterised in that most of the excess diisocyanate monomer and of the solvent when the latter is present are removed before the extraction with carbon dioxide in the liquid or supercritical state.